# FASCICULE DE BREVET EUROPEEN

(11) **EP 1 514 534 B1**
(45) Date de publication et mention de la délivrance du brevet: **28.02.2007**
(21) Numéro de dépôt: 04292084.3
(22) Date de dépôt: 24.08.2004
(51) Int. Cl.: A61K 8/02, A61K 8/19, A61Q 19/00, A61Q 5/00, A61Q 1/04, A61Q 1/14

(54) **Composition biphase et ses utilisations dans le domaine cosmetique**
Biphasiches Mittel und dessen Verwendungen in Kosmetika
Biphasic composition and its uses in cosmetics

(30) Priorité: 12.09.2003 FR 0310748
(43) Date de publication de la demande: 16.03.2005
(73) Titulaire: L'ORÉAL, 75008 Paris (FR)
(72) Inventeur: De Salvert, Armelle, 75013 Paris (FR); Bonafos, Arnaud, 75014 Paris (FR)
(74) Mandataire: Rasson, Catherine

(56) Documents cités:
- EP-A- 0 175 485
- EP-A- 0 603 080
- WO-A-00/61716
- WO-A-02/15849
- DE-A- 10 060 095
- US-B1- 6 689 223

## Description

La présente invention a pour objet une composition pour application topique, constituée de deux phases distinctes, une phase aqueuse et une phase huileuse, s'émulsionnant facilement par agitation et se déphasant facilement après arrêt de l'agitation. La présente invention a aussi pour objet l'utilisation de la dite composition dans le domaine cosmétique ou dermatologique, et notamment pour le démaquillage, le nettoyage et/ou le soin de la peau, des lèvres et/ou des yeux, et/ou pour le soin des cheveux.

Les compositions de ce type constituées de deux phases distinctes, notamment d'une phase aqueuse et d'une phase huileuse distinctes et non émulsionnées l'une dans l'autre au repos, sont généralement désignées sous le terme de "composition biphase". Elles se distinguent des émulsions par le fait qu'au repos, les deux phases sont distinctes au lieu d'être émulsionnées l'une dans l'autre. L'utilisation de ces compositions biphases nécessite une agitation préalable afin de former une émulsion, celle-ci devant être de qualité et de stabilité suffisantes pour permettre une application homogène des deux phases sur la peau ou la matière kératinique où elle est appliquée. Au repos, lesdites phases doivent se séparer rapidement et retrouver leur état initial, ce phénomène étant plus connu sous le terme de "déphasage".

Des compositions biphases ont déjà été décrites, par exemple dans les documents EP-A-370856 et EP-A-603080, notamment pour le démaquillage des yeux.

Un déphasage (ou démixage) rapide des deux phases après leur utilisation constitue une des qualités recherchées des compositions biphases. En effet, l'obtention d'un déphasage rapide est souhaitable pour diverses raisons, notamment parce qu'une mauvaise séparation des deux phases est perçue comme étant inesthétique par les utilisateurs. Le document EP-A-603080 décrit l'utilisation d'alkyldiméthylbenzylammonium et notamment du chlorure de benzalkonium comme agent de déphasage, pour obtenir ce déphasage rapide. Toutefois, on cherche à diversifier le type de molécule utilisée comme agent de déphasage pour avoir à disposition des compositions plus variées. Mais il s'est avéré difficile de trouver des agents de déphasage qui permettent d'obtenir un bon déphasage sans que ne se produise la formation de mousse dans la phase huileuse du biphase lors de l'agitation. Or, cette formation de mousse est rédhibitoire pour l'utilisateur.

Il subsiste donc le besoin d'une composition biphase constituée de deux phases distinctes non miscibles, qui, après agitation et formation extemporanée et éphémère de l'émulsion, ait un déphasage rapide en deux phases, sans formation de mousse.

De manière surprenante, la demanderesse a trouvé que l'utilisation du bicarbonate de sodium permettait d'obtenir une composition biphase ayant les qualités recherchées, c'est-à-dire se séparant rapidement en deux phases limpides après utilisation, sans qu'il n'y ait formation de mousse dans la phase huileuse lors de l'agitation. Ainsi, la présence de bicarbonate de sodium permet d'obtenir un bon déphasage des deux phases après agitation, tout en évitant la formation de mousse dans la phase huileuse lors de l'agitation. En outre, ce composé présente l'avantage d'être très bien toléré, y compris pour une application sur les yeux qui sont particulièrement sensibles aux composés susceptibles d'être agressifs. Le bicarbonate de sodium ne présente aucune agressivité et peut donc être avantageusement utilisé dans le domaine cosmétique et en particulier dans une composition de démaquillage des yeux.

Plus particulièrement, l'invention a pour objet une composition pour application topique, constituée d'une phase aqueuse et d'une phase huileuse distinctes, en un rapport pondéral allant de 25/75 à 90/10, caractérisée par le fait qu'elle contient du bicarbonate de sodium.

La composition selon l'invention étant destinée à une application topique, elle contient un milieu physiologiquement acceptable, c'est-à-dire compatible avec la peau, les muqueuses, les cheveux et le cuir chevelu.

La composition selon l'invention comprend au moins une phase aqueuse et une phase huileuse distinctes. Ces deux phases sont distinctes, c'est-à-dire qu'elles sont visibles l'une au-dessus de l'autre au repos. En outre, elles sont transparentes au repos. Les deux phases peuvent être ou non colorées.

Le mot « transparent » signifie que la composition a une turbidité inférieure ou égale à 300 NTU. La transparence d'une composition peut se mesurer par sa turbidité, et les NTU (Nephelometric Turbidity Units) sont les unités de mesure de la turbidité d'une composition. La mesure de turbidité peut être faite par exemple avec un turbidimètre model 2100P de la société HACH Compagny, les tubes utilisés pour la mesure étant référencés AR397A cat 24347-06. Les mesures sont effectuées à température ambiante (20°C à 25°C). La composition de l'invention a une turbidité allant généralement de 2 à 300 NTU, et de préférence de 5 à 200 NTU.

Le bicarbonate de sodium est le sel de sodium NaHCO₃, et il est d'autant plus surprenant qu'il permette un bon déphasage des compositions biphases que les autres sels et notamment les autres sels de sodium ne permettent pas d'atteindre le but recherché comme le montreront plus loin les exemples comparatifs. La quantité de bicarbonate de sodium peut aller par exemple de 0,005 à 5 % en poids, de préférence de 0,01 à 5 % en poids, mieux de 0,05 à 3 % en poids et encore mieux de 0,1 à 2 % en poids par rapport au poids total de la composition.

Il est possible d'ajouter en plus du bicarbonate, un autre sel tel que par exemple le chlorure de sodium, qui n'a aucun effet sur le déphasage mais qui est utilisé comme adjuvant dans une composition de démaquillage des yeux pour que la composition ait une pression osmotique proche de celle des larmes. La quantité de sel(s) autres que le bicarbonate peut aller par exemple de 0 à 5 % en poids et de préférence de 0,001 à 3 % en poids et mieux de 0,005 à 2 % en poids par rapport au poids total de la composition.

Le rapport pondéral entre la phase aqueuse et la phase huileuse va de 25/75 à 90/10, de préférence 30/70 à 70/30 et mieux de 40/60 à 60/40. Ainsi, la phase aqueuse représente généralement de 25 à 90 % en poids, de préférence de 30 à 70 % en poids et mieux de 40 à 60 % en poids par rapport au poids total de la composition.

La phase aqueuse de la composition selon l'invention comprend de l'eau et tout additif hydrosoluble ou hydrodispersible. L'eau utilisée peut être de l'eau déminéralisée stérile et/ou une eau florale telle que de l'eau de rose, de l'eau de bleuet, de l'eau de camomille ou de l'eau de tilleul, et/ou une eau thermale ou minérale naturelle, comme par exemple : l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau de Néris-les-Bains, l'eau d'Allevar-les-Bains, l'eau de Digne, l'eau de Maizières, l'eau de Neyrac-les-Bains, l'eau de Lons-le-Saunier, les Eaux Bonnes, l'eau de Rochefort, l'eau de Saint Christau, l'eau des Fumades et l'eau de Tercis-les-bains, l'eau d'Avene. La phase aqueuse peut comprendre aussi de l'eau thermale reconstituée, c'est-à-dire une eau contenant des oligoéléments tels que zinc, cuivre, magnésium, etc.., reconstituant les caractéristiques d'une eau thermale.

Comme additifs hydrosolubles, on peut citer notamment les polyols tels que la glycérine et les glycols tels que l'hexylèneglycol, les polyéthylèneglycols et le polypropylèneglycol. Les polyols peuvent être présents en une quantité allant de 0 à 5 % en poids, de préférence de 0,01 et 5 %, mieux de 0,05 à 3 % en poids et encore mieux de 0,1 à 3 % en poids par rapport au poids total de la composition. Selon un mode préféré de réalisation de l'invention, la composition contient au moins un polyol, de préférence la glycérine ou l'hexylène glycol ou leurs mélanges.

Comme additifs hydrosolubles, on peut citer aussi les alcools primaires en C₂-C₈, et notamment l'éthanol. Selon un mode particulier de réalisation de l'invention, la composition est de préférence pratiquement exempte d'éthanol. On entend ici par "pratiquement exempte d'éthanol", une composition contenant moins de 2 % en poids et de préférence moins de 1 % en poids d'éthanol par rapport au poids total de la composition.

La phase huileuse représente généralement de 10 à 75 %, de préférence de 30 à 70 % en poids, et mieux de 40 à 60 % en poids par rapport au poids total de la composition.

La phase huileuse de la composition selon l'invention peut être constituée d'une ou plusieurs huiles, celles-ci pouvant être des huiles minérales, végétales ou synthétiques ou encore des huiles de silicone. Elle peut comprendre en outre des additifs liposolubles ou lipodispersibles.

Selon un mode préféré de réalisation de l'invention, la phase huileuse comprend une ou plusieurs huiles choisies parmi les huiles hydrocarbonées d'origine minérale ou synthétique et les huiles de silicone. Plus particulièrement, la phase huileuse contient avantageusement une ou plusieurs huiles volatiles choisies parmi les huiles hydrocarbonées volatiles d'origine minérale ou synthétique et les huiles de silicone volatiles.

Par huile hydrocarbonée, on entend une huile formée essentiellement, voire constituée, d'atomes de carbone et d'hydrogène, et éventuellement d'atomes d'oxygène, d'azote, et ne contenant pas d'atome de silicium ou de fluor; elle peut contenir des groupes ester, éther, amine, amide.

Comme huiles hydrocarbonées volatiles d'origine minérale ou synthétique, on peut citer les iso-alcanes (appelées aussi isoparaffines) en C₈-C₁₆, tels que l'isododécane, l'isodécane, l'isohexadécane, comme par exemple les iso-alcanes vendus sous les noms commerciaux Isopar par la société Exxon Chemical ou les huiles vendues sous les noms commerciaux Permethyl par la société Presperse ; et leurs mélanges.

Comme huiles hydrocarbonées non-volatiles d'origine minérale ou synthétique, on peut citer l'huile de vaseline, le polyisobutène hydrogéné tel que l'huile de Parléam® ; et leurs mélanges.

Par huile de silicone, on entend une huile contenant au moins un atome de silicium, et notamment contenant des groupes Si-O. L'huile de silicone peut être choisie parmi les huiles de silicone non volatiles, les huiles de silicone volatiles et leurs mélanges.

Les huiles de silicone volatiles utilisables dans l'invention peuvent être choisies parmi les huiles de silicone ayant un point éclair allant de 40°C à 102°C, de préférence ayant un point éclair supérieur à 55°C et inférieur ou égal à 95°C, et préférentiellement allant de 65°C à 95°C. Comme huiles de silicone volatiles, on peut citer les huiles de silicones linéaires ou cycliques ayant de 2 à 7 atomes de silicium, ces silicones comportant éventuellement des groupes alkyle ou alkoxy ayant de 1 à 10 atomes de carbone. Comme exemples d'huiles de silicone volatiles, on peut citer notamment les cyclopolydiméthylsiloxanes (nom INCI : cyclomethicone), telles que le cyclopentasiloxane, le cyclohexasiloxane, l'octaméthylcyclotétrasiloxane, le décaméthylcyclopentasiloxane, le dodécaméthylcyclohexasiloxane ; les silicones linéaires telles que l'heptaméthylhexyltrisiloxane, l'heptaméthyloctyl-trisiloxane, l'hexaméthyl-disiloxane, l'octaméthyltrisiloxane, le décaméthyltétrasiloxane, le dodécaméthyl pentasiloxane ; et leurs mélanges.

L'huile de silicone non volatile utilisable dans l'invention peut être choisie parmi les polydiméthylsiloxanes (PDMS), et les polyméthylsiloxanes phénylés tels que les phényltriméthicones, les phényldiméthicones, les phényltriméthylsiloxydiphénylsiloxanes, les diphényl-diméthicones, les diphénylméthyldiphényl trisiloxanes, les 2-phényléthyltriméthyl-siloxysilicates, et les polyméthylphénylsiloxanes ; les polysiloxanes modifiés par des acides gras, des alcools gras ou des polyoxyalkylènes, et leurs mélanges.

De manière préférée, la phase huileuse contient au moins une huile volatile et notamment au moins un iso-alcane, de préférence un mélange d'isododécane et d'isohexadécane. Selon un autre mode préférentiel de réalisation de l'invention, la phase huileuse contient au moins un iso-alcane et au moins une huile de silicone volatile, et de préférence un mélange d'un ou plusieurs iso-alcanes choisis parmi l'isododécane et l'isohexadécane, et d'une ou plusieurs huiles de silicone volatiles.

Par ailleurs, la phase huileuse peut contenir une ou plusieurs autres huiles volatiles ou non, choisies parmi les huiles hydrocarbonées d'origine animale ou végétale, les esters et éthers de synthèse, les alcools gras, les huiles fluorées et leurs mélanges.

Par huile fluorée, on entend une huile contenant au moins un atome de fluor.

On peut citer par exemple comme huiles utilisables dans la composition de l'invention :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'amande d'abricot, de macadamia, d'arara, de coriandre, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité ;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrytyle.

Les huiles peuvent éventuellement être constituées uniquement d'huiles volatiles.

La composition biphase selon l'invention peut comprendre éventuellement un ou plusieurs tensioactifs dans l'une ou l'autre des phases, notamment quand elle est utilisée comme composition de démaquillage ou de nettoyage, car la présence d'un tensioactif permet à la fois d'obtenir un bon démaquillage des compositions de maquillage et notamment des mascaras, et aussi d'avoir une absence de sensation grasse lors du démaquillage. Toutefois, la composition de l'invention peut aussi être exempte de tensioactif, et si elle en contient, la quantité de tensioactif doit être telle que la composition reste au repos sous forme de deux phases distinctes et non pas sous forme d'émulsion.

Quand la composition de l'invention contient un tensioactif, celui-ci peut être du type anionique, non ionique ou amphotère, mais il est de préférence du type non ionique, et il est de préférence présent dans la phase aqueuse de la composition.

La quantité de tensioactif(s) en matière active peut aller par exemple de 0,001 à 1 % en poids, de préférence de 0,002 à 0,5 % en poids et mieux de 0,01 à 0,3 % en poids par rapport au poids total de la composition.

Parmi les agents tensioactifs non ioniques, ceux particulièrement préférés sont :
- les esters gras de sorbitol polyoxyéthylénés tels que le produit vendu sous la dénomination TWEEN 20 par la Société ICI ;
- les condensats d'oxyde d'éthylène et d'oxyde de propylène tels que ceux vendus sous les dénominations SYNPERONIC PE par la Société ICI et en particulier ceux référencés L 31, L 64, F 38, F 88, L 92, P 103, F 108 et F 127 ;
- les alkylpolyglycosides tels que ceux de formule générale (I) suivante :

   R-O-(G)ₓ (I)

   dans laquelle R représente un radical alkyle linéaire ou ramifié, saturé ou insaturé, comportant de 6 à 30 atomes de carbone, G représente un sucre réduit comportant de 5 à 6 atomes de carbone, et x désigne une valeur allant de 1 à 15 ;
- et leurs mélanges.

Selon un mode préféré de réalisation de l'invention, quand la composition contient un tensioactif, elle contient au moins un tensioactif non ionique et de préférence au moins un alkylpolyglycoside.

Les alkylpolyglycosides utilisés de préférence dans la composition selon la présente invention sont des composés de formule (I) dans laquelle R désigne plus particulièrement un radical alkyle comportant de 6 à 30 atomes de carbone et de préférence de 8 à 16 atomes de carbone, G désigne le glucose, le fructose ou le galactose, x est une valeur allant 1 à 4 et plus particulièrement de 1 à 3. Selon un mode préféré de réalisation de l'invention, l'alkylpolyglycoside utilisé dans la composition de l'invention est un alkylpolyglucoside, c'est-à-dire un composé de formule (I) dans laquelle G désigne le glucose et x est une valeur allant de 1,2 à 3.

Comme alkylpolyglucosides, on peut citer par exemple le decylglucoside (Alkyl-C9/C11-polyglucoside (1.4)) comme le produit commercialisé sous la dénomination MYDOL 10 par la société Kao Chemicals, le produit commercialisé sous la dénomination PLANTAREN 2000 UP et PLANTACARE 2000 UP par la société Henkel, et le produit commercialisé sous la dénomination ORAMIX NS 10 par la société Seppic ; le caprylyl/capryl glucoside comme le produit commercialisé sous la dénomination ORAMIX CG 110 par la Société Seppic ou sous la dénomination LUTENSOL GD 70 par la Société BASF ; le laurylglucoside comme les produits commercialisés sous les dénominations PLANTAREN 1200 N et PLANTACARE 1200 par la société Henkel ; et le coco-glucoside comme le produit commercialisé sous la dénomination PLANTACARE 818/UP par la société Henkel, et leurs mélanges.

La composition selon l'invention peut également contenir des adjuvants ou additifs cosmétiques conventionnels qui se trouveront dans l'une ou l'autre phase selon leur nature hydrophile ou lipophile, tels que par exemple des parfums, des agents conservateurs et bactéricides, des colorants, des agents adoucissants, des tampons, des humectants, des filtres U.V. (ou filtres solaires), des électrolytes tel que le chlorure de sodium comme indiqué plus haut, ou un ajusteur de pH (par exemple acide citrique ou hydroxyde de sodium), et leurs mélanges.

Comme conservateurs, on peut utiliser tout conservateur habituellement utilisé dans les domaines considérés, tels que par exemple les parabens, le gluconate de chlorhexidine et le chlorhydrate de polyhexaméthylène biguanide (nom CTFA Polyaminopropyl biguanide). Selon un mode préféré de réalisation de l'invention, la composition contient du chlorhydrate de polyhexaméthylène biguanide, seul ou en mélange avec d'autres conservateurs.

Comme bactéricide, on peut par exemple utiliser un mono(C₃-C₉)alkyl-ou (C₃-C₉)alcényléther de glycérol dont la fabrication est décrite dans la littérature, en particulier dans E. Baer, H.O.L. Fischer - J. Biol. Chem. 140-397-1941. Parmi ces mono(C₃-C₉)alkyl-ou (C₃-C₉)alcényléthers de glycérol, on utilise de préférence le 3-[(2-ethylhexyl)oxy]-1,2-propanediol, le 3-[(heptyl)oxy]-1,2-propanediol, le 3-[(octyl)oxy]-1,2-propanediol et le 3-[(allyl)oxy]-1,2-propanediol. Un mono(C₃-C₉)alkyléther de glycérol plus particulièrement préféré selon la présente invention est le 3-[(2-ethylhexyl)oxy]-1,2-propanediol, vendu par la société SCHULKE & MAYR G.m.b.H. sous la dénomination commerciale SENSIVA SC 50 (nom INCI : Ethylhexylglycerin).

Parmi les agents adoucissants, on peut en particulier citer l'allantoïne, le bisabollol, les planctons, et certains extraits de plantes comme les extraits de rose et les extraits de mélilot.

Le ou les actifs pouvant être présents dépendent du but final de la composition. Comme actifs utilisables dans la composition de l'invention, notamment quand il s'agit d'une composition de soin de la peau, on peut citer par exemple, les enzymes (par exemple lactoperoxydase, lipase, protéase, phospholipase, cellulases) ; les flavonoïdes tels que les isoflavones ; les agents hydratants tels que les hydrolysats de protéines ; le hyaluronate de sodium ; les anti-inflammatoires ; les oligomères procyannidoliques ; les vitamines comme la vitamine A (rétinol), la vitamine E (tocophérol), la vitamine C (acide ascorbique), la vitamine B5 (panthénol), la vitamine B3 (niacinamide), les dérivés de ces vitamines (notamment esters) et leurs mélanges ; l'urée ; la caféine ; les dépigmentants tels que l'acide kojique, l'hydroquinone et l'acide caféique ; l'acide salicylique et ses dérivés ; les alpha-hydroxyacides tels que l'acide lactique et l'acide glycolique et leurs dérivés ; les rétinoïdes tels que les caroténoïdes et les dérivés de vitamine A ; l'hydrocortisone ; la mélatonine ; les extraits d'algues, de champignons, de végétaux, de levures, de bactéries ; les stéroïdes ; les actifs anti-bactériens comme le 2,4,4'-trichloro-2'-hydroxy diphényl éther (ou triclosan), le 3,4,4'-trichlorocarbanilide (ou triclocarban) et les acides indiqués ci-dessus et notamment l'acide salicylique et ses dérivés ; les agents tenseurs ; les céramides ; les huiles essentielles ; et leurs mélanges ; et tout actif approprié pour le but final de la composition.

Les filtres U.V. peuvent être présents dans la composition selon l'invention, notamment quand elle est destinée à une protection solaire. Ces filtres peuvent être notamment des filtres organiques, et ils peuvent être présents en une quantité en matière active allant de 0,01 à 20 % en poids de matière active, de préférence de 0,1 à 15 % en poids, et mieux 0,2 à 10 % en poids par rapport au poids total de la composition.

Comme exemples de filtres organiques, actifs dans l'UV-A et/ou l'UV-B, pouvant être ajoutés dans la composition de l'invention, on peut citer par exemple, les dérivés à fonction sulfonique tels que les dérivés sulfonés ou sulfonatés du benzylidène camphre, de la benzophénone ou du phénylbenzimidazole, plus particulièrement les dérivés du benzylidène camphre, comme l'acide benzène 1,4 [di(3-méthylidène-campho 10-sulfonique)], (nom INCI : Terephthalylidene Dicamphor Sulfonic Acid) fabriqué sous le nom « MEXORYL SX » par la société CHIMEX. l'acide 4'-sulfo 3-benzylidènecamphre (nom INCI : Benzylidene Camphor Sulfonic Acid), fabriqué sous le nom « MEXORYL SL» par la société CHIMEX, l'acide 2-[4-(camphométhylidène) phényl] benzimidazole-5-sulfonique, l'acide phénylbenzimidazole sulfonique (nom INCI : Phenylbenzimidazole Sulfonic Acid), commercialisé sous le nom EUSOLEX 232 par la société MERCK ; les dérivés de l'acide para-aminobenzoique ; les dérivés salicyliques tels que le salicylate d'éthyl hexyle vendu sous le nom commercial NEO HELIOPAN OS par Haarmann et Reimer ; les dérivés du dibenzoylméthane tels que le Butyl Methoxydibenzoylmethane vendu notamment sous le nom commercial PARSOL 1789 par Hoffmann La Roche ; les dérivés cinnamiques tels que l'éthylhexyl Methoxycinnamate vendu notamment sous le nom commercial PARSOL MCX par Hoffmann La Roche ; les dérivés de β,β'-diphénylacrylate tels que l'octocrylene (α-cyano-β,β-diphénylacrylate de 2-éthylhexyle) vendu sous le nom commercial UVINUL N539 par la société BASF ; les dérivés de la benzophénone tels que la Benzophenone-1 vendu sous le nom commercial UVINUL 400 par BASF, la Benzophenone-2 vendu sous le nom commercial UVINUL D50 par BASF, la Benzophenone-3 ou Oxybenzone, vendu sous le nom commercial UVINUL M40 par BASF, la Benzophenone-4 vendu sous le nom commercial UVINUL MS40 par BASF ; les Dérivés du benzylidène camphre tels que le 4-Methylbenzylidene camphre vendu sous le nom commercial EUSOLEX 6300 par MERCK ; les dérivés du phenyl benzimidazole tels que le Benzimidazilate vendu sous le nom commercial NEO HELIOPAN AP par Haarmann et Reimer ; les dérivés de la triazine tels que l'Anisotriazine vendu sous le nom commercial TINOSORB S par CIBA GEIGY et l'éthylhexyl triazone vendu notamment sous le nom commercial UVINUL T150 par BASF ; les dérivés du phenyl benzotriazole tels que Drometrizole Trisiloxane vendu sous le nom commercial SILATRIZOLE par Rhodia Chimie ; les dérivés anthraniliques tels que le Menthyl anthranilate vendu sous le nom commercial NEO HELIOPAN MA par Haarmann et Reimer ; les dérivés d'imidazolines ; les dérivés du benzalmalonate ; et leurs mélanges.

Les compositions décrites ci-dessus peuvent être conditionnées, de façon connue, dans un flacon à un seul compartiment. L'utilisateur doit alors agiter le flacon avant d'en verser le contenu sur un coton. On peut également prévoir que les deux phases de la composition soient introduites dans deux compartiments indépendants d'un même flacon, un système étant prévu pour leur mélange au moment de la distribution. De tels dispositifs sont décrits par exemple dans les documents EP-A-497256 et FR-A-2697233.

La composition selon l'invention peut être utilisée pour toute application topique ; notamment, elle peut constituer une composition cosmétique ou dermatologique.

Elle peut en particulier être utilisée pour le soin, le nettoyage et/ou le démaquillage de la peau, les lèvres et /ou des yeux, et également comme composition pour le soin des cheveux.

L'invention a aussi pour objet l'utilisation cosmétique d'une composition cosmétique telle que définie ci-dessus, pour le soin, le démaquillage et/ou le nettoyage de la peau, des lèvres et/ou des yeux, et/ou pour le soin des cheveux.

La présente invention a encore pour objet un procédé cosmétique de démaquillage, de nettoyage et/ou de soin de la peau, des lèvres et/ou des yeux, caractérisé par le fait que l'on applique sur la peau, les lèvres et/ou les yeux, une composition cosmétique telle que définie ci-dessus.

La présente invention a aussi pour objet un procédé cosmétique de soin des cheveux, caractérisé par le fait que l'on applique sur les cheveux, une composition cosmétique telle que définie ci-dessus.

Selon un mode préféré de réalisation de l'invention, la composition constitue une composition de démaquillage des yeux.

L'exemple ci-après de compositions selon l'invention est donné à titre d'illustration et sans caractère limitatif. Les quantités y sont données en % en poids, sauf mention contraire.

### Exemple 1 : composition démaquillante

### Phase huileuse

- Cyclopentasiloxane 35 %
- Isododécane 7,5 %
- isohexadécane 7,5 %
- Colorant 0,05 %

### Phase aqueuse

- Glycérine 1,5 %
- Chlorhydrate de polyhexaméthylène biguanide 0,35 %
- Bicarbonate de sodium 1 %
- Eau déminéralisée 47,10 %

Mode opératoire : On mélange les constituants de la phase huileuse d'une part et ceux de la phase aqueuse d'autre part. Puis, on agite les deux phases.

On obtient une composition qui, au repos, comporte une phase aqueuse et une phase huileuse distinctes. Quand on les agite, l'émulsion se forme et aucune mousse n'apparaît. Après l'arrêt de l'agitation, les deux phases se séparent rapidement.

### Exemple 2 : composition de soin de la peau

### Phase huileuse

- Cyclopentasiloxane 30 %
- Polyisobutène hydrogéné 19,5 %
- Huile d'amande d'abricot (Prunus Armeniaca kernel Oil) 0,5 %

### Phase aqueuse

- Glycérine 1 %
- Chlorhydrate de polyhexaméthylène biguanide 0,35 %
- Bicarbonate de sodium 1 %
- Eau déminéralisée 47,65 %

Mode opératoire : On mélange les constituants de la phase huileuse d'une part et ceux de la phase aqueuse d'autre part. Puis on agite les deux phases.

Cette composition peut être utilisée notamment pour le soin de la peau.

### Exemple 1 comparatif : composition démaquillante

On remplace dans l'exemple 1, le bicarbonate de sodium par du sulfate de sodium.

On obtient une composition comportant de la mousse dans la phase grasse.

### Exemple 2 comparatif : composition démaquillante

On remplace dans l'exemple 1, le bicarbonate de sodium par du lactate de sodium.

On obtient une composition comportant de la mousse dans la phase grasse.

## Revendications

1. Composition pour application topique, constituée d'une phase aqueuse et d'une phase huileuse distinctes, en un rapport pondéral allant de 25/75 à 90/10, **caractérisée par le fait qu**'elle contient du bicarbonate de sodium.

2. Composition selon la revendication précédente, **caractérisée en ce que** la quantité de bicarbonate va de 0,005 à 5 % en poids en poids par rapport au poids total de la composition.

3. Composition selon la revendication 1 ou 2, **caractérisée en ce que** le rapport pondéral entre la phase aqueuse et la phase huileuse va de 30/70 à 60/40.

4. Composition selon l'une quelconque des revendications précédentes, **caractérisée en ce que** la phase aqueuse représente de 25 à 90 % en poids par rapport au poids total de la composition et la phase huileuse représente de 10 à 75 % en poids par rapport au poids total de la composition.

5. Composition selon l'une quelconque des revendications précédentes, **caractérisée en ce que** la phase huileuse comprend une ou plusieurs huiles choisies parmi les huiles hydrocarbonées d'origine minérale ou synthétique et les huiles de silicone.

6. Composition selon la revendication précédente, **caractérisée en ce que** la phase huileuse contient au moins une huile volatile.

7. Composition selon la revendication précédente, **caractérisée en ce que** la phase huileuse contient un mélange d'un ou plusieurs iso-alcanes choisis parmi l'isododécane et l'isohexadécane, et d'une ou plusieurs huiles de silicone volatiles.

8. Composition selon l'une quelconque des revendications précédentes, **caractérisée en ce qu**'elle comprend au moins un tensioactif choisi parmi les tensioactifs non ioniques.

9. Composition selon l'une quelconque des revendications précédentes, **caractérisée en ce que** le tensioactif est présent en une quantité allant de 0,001 à 1 % en poids par rapport au poids total de la composition.

10. Composition selon l'une quelconque des revendications précédentes, **caractérisée en ce qu**'elle constitue une composition cosmétique ou dermatologique.

11. Utilisation cosmétique d'une composition cosmétique selon l'une quelconque des revendications 1 à 9, pour le soin, le démaquillage et/ou le nettoyage de la peau, des lèvres et/ou des yeux et/ou pour le soin des cheveux.

12. Procédé cosmétique de démaquillage, de nettoyage et/ou de soin de la peau, des lèvres et/ou des yeux, **caractérisé par le fait que** l'on applique sur la peau, les lèvres et/ou les yeux, une composition cosmétique selon l'une quelconque des revendications 1 à 9.

13. Procédé cosmétique de soin des cheveux, **caractérisé par le fait que** l'on applique sur les cheveux, une composition cosmétique selon l'une quelconque des revendications 1 à 9.

## Claims

1. Composition for topical application, consisting of an aqueous phase and of a separate oily phase, in a weight ratio ranging from 25/75 to 90/10, **characterized in that** it contains sodium bicarbonate.

2. Composition according to the preceding claim, **characterized in that** the amount of bicarbonate ranges from 0.005% to 5% by weight relative to the total weight of the composition.

3. Composition according to Claim 1 or 2, **characterized in that** the weight ratio between the aqueous phase and the oily phase ranges from 30/70 to 60/40.

4. Composition according to any one of the preceding claims, **characterized in that** the aqueous phase represents from 25% to 90% by weight relative to the total weight of the composition and the oily phase represents from 10% to 75% by weight relative to the total weight of the composition.

5. Composition according to any one of the preceding claims, **characterized in that** the oily phase comprises one or more oils chosen from hydrocarbon-based oils of mineral or synthetic origin and silicone oils.

6. Composition according to the preceding claim, **characterized in that** the oily phase contains at least one volatile oil.

7. Composition according to the preceding claim, **characterized in that** the oily phase contains a mixture of one or more isoalkanes chosen from isododecane and isohexadecane, and one or more volatile silicone oils.

8. Composition according to any one of the preceding claims, **characterized in that** it comprises at least one surfactant chosen from nonionic surfactants.

9. Composition according to any one of the preceding claims, **characterized in that** the surfactant is present in an amount ranging from 0.001% to 1% by weight relative to the total weight of the composition.

10. Composition according to any one of the preceding claims, **characterized in that** it is a cosmetic or dermatological composition.

11. Cosmetic use of a cosmetic composition according to any one of Claims 1 to 9, for caring for, removing makeup from and/or cleansing the skin, the lips and/or the eyes, and/or for haircare.

12. Cosmetic process for removing makeup from, cleansing and/or caring for the skin, the lips and/or the eyes, **characterized in that** a cosmetic composition according to any one of Claims 1 to 9 is applied to the skin, the lips and/or the eyes.

13. Cosmetic haircare process, **characterized in that** a cosmetic composition according to any one of Claims 1 to 9 is applied to the hair

## Patentansprüche

1. Zusammensetzung für die topische Anwendung, die aus einer wässrigen Phase und einer davon getrennt vorliegenden Ölphase in einem Gewichtsverhältnis besteht, das im Bereich von 25/75 bis 90/10 liegt, **dadurch gekennzeichnet, dass** sie Natriumhydrogencarbonat enthält.

2. Zusammensetzung nach dem vorhergehenden Anspruch, **dadurch gekennzeichnet, dass** die Menge an Hydrogencarbonat im Bereich von 0,005 bis 5 Gew.-%, bezogen auf das Gesamtgewicht der Zusammensetzung, liegt.

3. Zusammensetzung nach Anspruch 1 oder 2, **dadurch gekennzeichnet, dass** das Gewichtsverhältnis zwischen der wässrigen Phase und der Ölphase im Bereich von 30/70 bis 60/40 liegt.

4. Zusammensetzung nach einem der vorhergehenden Ansprüche, **dadurch gekennzeichnet, dass** die wässrige Phase 25 bis 90 Gew.-%, bezogen auf das Gesamtgewicht der Zusammensetzung, und die Ölphase 10 bis 75 Gew.-%, bezogen auf das Gesamtgewicht der Zusammensetzung, ausmacht.

5. Zusammensetzung nach einem der vorhergehenden Ansprüche, **dadurch gekennzeichnet, dass** die Ölphase ein oder mehrere Öle enthält, die unter den Kohlenwasserstoffölen mineralischer oder synthetischer Herkunft und den Siliconölen ausgewählt werden.

6. Zusammensetzung nach dem vorhergehenden Anspruch, **dadurch gekennzeichnet, dass** die Ölphase mindestens ein flüchtiges Öl enthält.

7. Zusammensetzung nach dem vorhergehenden Anspruch, **dadurch gekennzeichnet, dass** die Ölphase ein Gemisch aus einem oder mehreren Isoalkanen, die unter Isododecan und Isohexadecan ausgewählt werden, und einem oder mehreren flüchtigen Siliconölen enthält.

8. Zusammensetzung nach einem der vorhergehenden Ansprüche, **dadurch gekennzeichnet, dass** sie mindestens einen grenzflächenaktiven Stoff enthält, der unter den nichtionischen grenzflächenaktiven Stoffen ausgewählt wird.

9. Zusammensetzung nach einem der vorhergehenden Ansprüche, **dadurch gekennzeichnet, dass** der grenzflächenaktive Stoff in einer Menge enthalten ist, die im Bereich von 0,001 bis 1 Gew.-%, bezogen auf das Gesamtgewicht der Zusammensetzung, liegt.

10. Zusammensetzung nach einem der vorhergehenden Ansprüche, **dadurch gekennzeichnet, dass** sie eine kosmetische oder dermatologische Zusammensetzung darstellt.

11. Kosmetische Verwendung einer kosmetischen Zusammensetzung nach einem der Ansprüche 1 bis 9 zum Pflegen, Abschminken und/oder Reinigen der Haut, der Lippen und/oder der Augen und/oder zum Pflegen der Haare.

12. Kosmetisches Verfahren zum Abschminken, Reinigen und/oder Pflegen der Haut, der Lippen und/ oder der Augen, **dadurch gekennzeichnet, dass** eine kosmetische Zusammensetzung nach einem der Ansprüche 1 bis 9 auf die Haut, die Lippen und/oder die Augen aufgetragen wird.

13. Kosmetisches Verfahren zum Pflegen der Haare, **dadurch gekennzeichnet, dass** eine kosmetische Zusammensetzung nach einem der Ansprüche 1 bis 9 auf die Haare aufgetragen wird.
